# EUROPÄISCHE PATENTSCHRIFT

(11) **EP 2 916 716 B1**
(45) Veröffentlichungstag und Bekanntmachung des Hinweises auf die Patenterteilung: **10.08.2022**
(21) Anmeldenummer: 13791952.8
(22) Anmeldetag: 01.11.2013
(51) Int. Cl.: A61B 5/00, A61B 5/026, A61B 5/1455

(54) **MESSVORRICHTUNG ZUR BESTIMMUNG ZEREBRALER PARAMETER**
MEASURING DEVICE FOR DETERMINING CEREBRAL PARAMETERS
DISPOSITIF DE MESURE POUR DÉTERMINER DES PARAMÈTRES CÉRÉBRAUX

(30) Priorität: 06.11.2012 CH 22662012
(43) Veröffentlichungstag der Anmeldung: 16.09.2015
(73) Patentinhaber: Luciole Medical AG, 8048 Zürich (CH)
(72) Erfinder: LÉCHOT, Christophe, 2503 Biel/Bienne (CH); FRÉLY, Jean-Claude, 2504 Biel (CH); AESCHLIMANN, Marcel, 2514 Ligerz (CH); FRÖHLICH, Jürg Hans, 8006 Zürich (CH); BAUMANN, Dirk, 8008 Zürich (CH); MUSER, Markus Hugo, 8820 Wädenswil (CH); OBERLE, Michael, 8006 Zürich (CH)
(74) Vertreter: BOVARD AG
(86) Internationale Anmeldenummer: PCT/EP2013/072870
(87) Internationale Veröffentlichungsnummer: WO 2014/072231

(56) Entgegenhaltungen:
- EP-A1- 0 481 612
- WO-A1-94/27494
- WO-A1-2009/062189
- WO-A2-2007/064984
- US-A- 5 090 410

## Beschreibung

Die vorliegende Erfindung betrifft eine Messvorrichtung und ein Verfahren zur Messung von Parametern eines Körpergewebes mit einer Sensoreinheit und einer Sensormatte zum Aufbringen auf das Körpergewebe, insbesondere eine nicht-invasive Messvorrichtung und ein nicht-invasives Verfahren zur Messung zerebraler Parameter, wie z. B. dem Sauerstoffgehalt des Gehirns.

### STAND DER TECHNIK

Es sind diverse nicht-invasive Verfahren zur zerebralen Diagnostik bekannt, bei welchen diverse zerebrale Parameter gemessen werden. Es werden beispielsweise Parameter bzgl. der Konzentration von desoxygeniertem und oxygeniertem Hämoglobin, des zerebralen Blutflusses oder des Gewebesauerstoffindexes gemessen. Zur Erfassung dieser Parameter kann z. B. eine Messvorrichtung auf einer Kopfoberfläche angeordnet, vorzugsweise auf der Stirn, und somit Messungen auf der Gehirnoberfläche durchgeführt werden. Zu derartigen Verfahren gehört z. B. die Nahinfrarotspektroskopie-Messung (NIRS).

Aus der EP 2294973 A2 ist beispielsweise ein Pulsoximetersensor bekannt, der eine ringförmige Kontaktauflage zur Auflage auf einer Körperoberfläche und einen Sensorkörper aufweist, der in einer Öffnung der Kontaktauflage aufgenommen ist. Der Sensorkörper ist fest mit der Kontaktauflage verbunden, kann jedoch relativ zu dieser bewegt, d. h. aufgeklappt, werden, um die Unterseite des Sensorkörpers zugänglich zu machen, wenn die Kontaktauflage auf der Körperoberfläche angebracht ist. Hierfür ist eine Scharnierverbindung zwischen der Kontaktauflage und dem Sensorkörper vorgesehen. Der Sensorkörper weist einen leicht flexiblen Optikhalter zur Aufnahme eines Emitters und eines Detektors auf, wobei der Detektor in einem festen Abstand zum Emitter angeordnet ist. Der Emitter und der Detektor sind mit einer Patientenkontaktfläche des Sensorkörpers verbunden. Auf der Patientenkontaktfläche sind mehrere gestapelte, einzeln abnehmbare Haftschichten angeordnet, die auf einer Körperfläche eines Patienten haften und nach einander verwendet werden. Dabei kann eine benutzte Haftschicht abgezogen und entsorgt werden, währen der Pulsoximetersensor mit der nächsten Haftschicht erneut auf der Körperoberfläche angebracht werden kann.

Weiter ist aus der US 7,047,054 eine wiederverwendbare NIRS-Überwachungsanordnung zur nicht-invasiven Überwachung des Blutsauerstoffgehalts bekannt, mit der das Energieniveau und die Grösse eines Laserfeldes auf einer Körperoberfläche kontrolliert und mit der das Rauschverhältnis zwischen Laserlicht und Detektor durch eine EMI-Abschirmung verbessert werden kann. Es werden Haftelemente eingesetzt, die an der wiederverwertbaren Überwachungsanordnung angeordnet werden, um die Überwachungsanordnung auf einer Körperoberfläche anzubringen. Die Haftelemente sind abnehmbar und werden nach einer Messung entsorgt.

Aus der WO 94/27494 ist eine spektralfotometrische Sensormatte bekannt, die z. B. als Oximeter verwendet wird. Die Sensormatte umfasst eine flexible Lage aus Schaummaterial zur Auflage auf einer Körperoberfläche und einen flexiblen Trägerrahmen, der Lichtquellen und Sensoren aufnehmen kann. Der Trägerrahmen bildet eine längliche Struktur, die in die flexible Lage integriert ist, wobei Bereich des Rahmens durch die flexible Lage hindurch treten. Die Sensormatte wird als Ganzes zur Messung auf die Körperoberfläche aufgebracht und muss auch als Ganzes wieder abgenommen werden.

Aus WO2009/062189 geht demnach eine Messvorrichtung für eine nicht-invasive Messung von Parametern hervor, welche eine Abdeckung, eine Sensoreinheit mit einer Aufnahme und einer Sensormatte zum lösbaren Aufbringen der Messvorrichtung auf einer Körperoberfläche aufweist, wobei Abdeckung, Sensoreinheit und Sensormatte voneinander lösbar sind.

Bei Messanordnungen nach dem Stand der Technik ist oftmals eine langwierige Reinigung erforderlich, um diese nach einer Messung für eine nächste Messung bereit zu stellen. Oder es werden Einweg-Anordnungen verwendet. Bei Messanordnungen mit mehreren Elementen, die bei einer Messung auf der Körperoberfläche zu liegen kommen sollen, kann es vorkommen, dass der Kontakt von Emittern und Detektoren auf der Körperoberfläche undefiniert oder ungleichmässig ist und somit die Messergebnisse nachteilig beeinträchtigt werden.

### AUFGABE DER ERFINDUNG

Es ist eine Aufgabe der vorliegenden Erfindung eine nicht-invasive Messvorrichtung und ein nicht-invasives Verfahren zur Messung von Parametern eines Körpergewebes bereit zu stellen, bei welchen die Handhabung der Vorrichtung und deren Konstruktion vereinfacht, die Kosten zur Durchführung von Messungen gesenkt und die Präzession von Messungen verbessert werden. Insbesondere soll ein homogener Kontakt zwischen der Messvorrichtung und einer Oberfläche des Körpergewebes sicher gestellt werden.

Diese und weitere Aufgaben werden von einer nicht-invasive Messvorrichtung und einem Verfahren zur Bereitstellung einer solchen Messvorrichtung gemäss der Erfindung gelöst. Das nicht-invasive Messverfahren der Erfindung ist in Anspruch 1 und das Verfahren zur Bereitstellung einer solchen Messvorrichtung ist in Anspruch 11 definiert. Besondere Ausgestaltungen und/oder Varianten gehen aus den Unteransprüchen hervor.

Eine Messvorrichtung zur nicht-invasiven Messung von Parametern eines Körpergewebes nach der vorliegenden Erfindung weist eine Abdeckung, eine Sensoreinheit und eine Sensormatte zum lösbaren Aufbringen, bzw. zum Befestigen, der Messvorrichtung, insbesondere der Sensoreinheit, auf einer Körperoberfläche auf. Abdeckung, Sensoreinheit und Sensormatte sind voneinander lösbar.

Die Sensoreinheit weist eine Aufnahme, z. B. aus Silikon, auf, die in ihrem Innenraum eine Sensoranordnung aufnimmt. Die Aufnahme ist beispielsweise als flaches, im wesentlichen längliches Gehäuse ausgebildet und weist an einer Unterseite eine Sensorfläche in Richtung der Körperoberfläche auf. In der Sensorfläche können ein oder mehrere Durchgänge oder Fenster vorgesehen sein, welche z. B. einen Lichtdurchgang in und aus der Aufnahme ermöglichen. Eine Oberseite der Aufnahme kann vollständig oder teilweise offen sein, um z. B. die Sensoranordnung darin zu montieren. Ist die Sensoranordnung innerhalb der Aufnahme untergebracht, kann die Oberseite verschlossen werden, z. B. mit einer Folie oder einem Deckel. Vorzugsweise wird eine lichtdichte Verschlussabdeckung verwendet, um eine Fremdlichteinstrahlung zu vermeiden. Die Verschlussabdeckung kann vorteilhafter Weise von einer Versiegelung gebildet werden, welche die Sensoranordnung im Innenraum der Aufnahme versiegelt. Hierfür kann z. B. schwarzes Silikon verwendet werden. Die Sensoreinheit bildet somit eine kompakte zu allen Seiten mit planen Flächen abgeschlossene Einheit. Die Sensoranordnung umfasst mehrere Elemente. Sie umfasst zumindest die Ausgänge einer oder mehrerer Lichtquellen, wie etwa von Laserdioden, oder eine oder mehrere Lichtquellen selbst, eine oder mehrere Messflächen, bzw. Sensoren, die vorzugsweise zu den Lichtquellen beabstandet vorgesehen sind, sowie optische, bzw. elektrische Leitungen, die z. B. Licht der Lichtquelle oder - quellen oder von den Messflächen empfangenes Licht oder elektrische Signale von und zu den Elementen der Sensoranordnung transportieren. Es können weitere Elemente in der Aufnahme vorgesehen werden, wie z. B. eine Kontrolleinheit, wie nachfolgend beschrieben wird. Vorzugsweise werden als Lichtquelle wenigstens vier verschiedene Laserdioden unterschiedlicher Wellenlänge vorgesehen, die zeitlich gestaffelt ein- und ausgeschaltet werden können. Die Elemente der Sensoranordnung sind vorzugsweise vollständig innerhalb der Aufnahme untergebracht, d. h. sie ragen nicht über die Aufnahme hinaus.

Die Sensormatte weist eine innere Aussparung oder einen Durchgang zum Aufnehmen der Sensoreinheit auf. Die Form der Aussparung, bzw. des Durchgangs, ist auf die äussere Form der Aufnahme abgestimmt. Die Aufnahme fügt sich somit mit ihrer Umfangsfläche passgenau in die Sensormatte ein und bildet mit dieser einen Formschluss. Ferner weist die Sensormatte eine zumindest teilweise umlaufende untere Auflagefläche zur Auflage auf der Körperoberfläche auf. Vorzugsweise umgibt die Auflagefläche der Sensormatte die Sensoreinheit vollständig und verläuft somit rings um die Sensoreinheit. Die Auflagefläche der Sensormatte ist im Bereich, in dem sie die Sensoreinheit umgibt, wenigstens 4 mm, vorzugsweise mindestens 5 mm breit, so dass sie seitlich vom Umfang der Aufnahme der Sensoreinheit um wenigstens diese 5 mm absteht.

Weiter ist die Abdeckung über eine obere Seite von Sensoreinheit und Sensormatte zum Verschliessen der Aussparung bzw. Durchführung während einer Messung von Parametern vorgesehen. Vorzugsweise deckt die Abdeckung die gesamte obere Seite der Sensormatte ab. Die Fläche der Abdeckung entspricht daher im Wesentlichen dem Ausmass der Oberseite der Sensormatte (8). Als Abdeckung dient z. B. ein geschlossenporiges Schaummaterial.

Die Sensoreinheit ist in der Sensormatte durch eine form- und/oder z.B. reibschlüssige Verbindung gehalten. Sie kann auch durch die Abdeckung auf der oberen Seite und einen Verschluss auf der unteren Seite in der Aussparung gehalten werden.

Gemäss der Erfindung sind die Sensoreinheit, die Sensormatte und die Abdeckung von einander lösbar vorgesehen. Das heisst, sie sind derart miteinander verbunden, dass zumindest die Sensoreinheit zerstörungsfrei von der Sensormatte und der Abdeckung gelöst werden kann. Die Verbindungs- oder Haltemittel zum Zusammenfügen von Sensoreinheit, Sensormatte und Abdeckung ermöglichen ein einfaches Entfernen der Abdeckung von Sensormatte und Sensoreinheit und Entnehmen der Sensoreinheit aus der Sensormatte. Hierfür sind Sensoreinheit, Sensormatte und Abdeckung als zusammen zu setzende Elemente eine Art Bausatz vorgesehen, die zur Bereitstellung der Messvorrichtung für eine Messung von Körperparametern z. B. von einer medizinischen Fachperson zusammengesetzt werden können. Dabei kann die Sensoreinheit als wiederverwendbare Einheit vorgesehen sein. Nach einer Messung kann sie gereinigt und für eine weitere Messung zur Verfügung gestellt werden. Die Sensormatte und die Abdeckung können als Einweg-Einheiten vorgesehen werden, d. h. nachdem sie nach einer Messung von der Körperoberfläche abgenommen werden, sollen sie entsorgt werden. Nach einer Messung können die Elemente des Bausatzes, d. h. die Sensormatte, die Sensoreinheit und die Abdeckung wieder voneinander gelöst, bzw. voneinander entfernt werden. Die Messvorrichtung der vorliegenden Erfindung kann daher kostengünstig zur Verfügung gestellt werden und kann in einfacher Weise unkomplizierter Weise für eine Parametermessung zur Verfügung gestellt werden.

Zum Anbringen der Messvorrichtung zur Messung von Parametern eines Körpergewebes auf der Körperoberfläche kann eine Haftlage über eine untere Seite von Sensoreinheit und Sensormatte vorgesehen sein. Die Haftlage ist vorteilhaft transparent ausgebildet, um einen Lichtdurchgang in und aus der Sensoreinheit zu gewährleisten. Die Haftlage stellt den Kontakt zwischen der Sensormatte und der Sensoreinheit sicher und bildet eine sterile Barriere zwischen der Körperoberfläche und der Messvorrichtung.

Da die Sensoreinheit in zusammengesetzten Zustand mit ihrer Oberseite gegen die Abdeckung stösst, wird die Sensoreinheit mit ihrer Unterseite, auf der sich die Ausgänge der Lichtquelle, bzw. -quellen, und die Messflächen befinden, gegen die Körperoberfläche gepresst. Dadurch wird ein guter Kontakt zwischen Körperoberfläche und Sensorfläche hergestellt. Ferner wird ein zuverlässiges Einstrahlen des ausgesandten Lichts in das Körpergewebe und Empfangen von zu messendem Licht an den Messflächen sichergestellt. Dadurch kann ein Signal zu Rausch Verhältnis verbessert und die Qualität der Messwerte der zu messenden Parameter erhöht werden. Dabei liegen die Sensorfläche und die Auflagefläche vorzugsweise in einer Ebene, um ein übermässiges Eindrücken der Sensoreinheit in die Körperoberfläche zu vermeiden. Dadurch wird das Körpergewebe durch die Sensoreinheit so wenig wie möglich beeinträchtigt und die Blutversorgung wird im Bereich der Messvorrichtung nicht beeinflusst.

In einer Ausführungsform der Messvorrichtung nach der vorliegenden Erfindung schlägt die Aufnahme an einer Innenseite der Abdeckung an, wie oben erwähnt. Demnach schliesst sich die Aufnahme nicht nur an die Abdeckung an, sondern die Abdeckung dient vielmehr auch als Anschlag und blockiert eine Bewegung der Sensoreinheit in Richtung der Abdeckung relativ zur Sensormatte. Die Sensormatte und die Aufnahme sind daher in montiertem Zustand der Messvorrichtung in dieser Richtung zu einander feststehend. In einem auf eine Körperoberfläche aufgebrachten Zustand der Messvorrichtung drückt die Abdeckung der Sensormatte die Aufnahme der Sensoreinheit und somit die Messflächen und die Ausgänge der Lichtquelle, bzw. -quellen, gegen die Körperoberfläche, wie oben erwähnt. Die Haftlage kann auf der Unterseite der Sensormatte als Fixierung der Sensoreinheit innerhalb der Aussparung dienen.

Vorzugsweise ist die Sensormatte flexibel, bzw. biegsam ausgebildet. Ober- und Unterseite, insbesondere die Auflagefläche, sind daher an eine Körperoberfläche anpassbar und können sich Erhebungen, Krümmungen oder Dellen in der Körperoberfläche anpassen. Die Sensormatte kann auch komprimierbar sein, so dass sich die Auflagefläche z. B. an Unebenheiten der Körperoberfläche eindrücken lässt. Die Sensormatte kann z. B. aus Schaum, wie etwa geschlossenporigem Schaummaterial oder Neopren, hergestellt sein. Schaum garantiert Biegsamkeit und bietet einen angenehmen Tragekomfort für einen Patienten. Ferner ist auch die Aufnahme er Sensoreinheit zumindest teilweise flexibel ausgebildet, so dass sich auch die Unterseite der Sensoreinheit an die Körperoberfläche anpassen kann. Durch die Flexibilität, bzw. Biegsamkeit, der Sensormatte und der Aufnahme wird der Kontakt zwischen der Messvorrichtung und der Körperoberfläche für die Übertragung von Licht und Messsignalen verbessert und der Tragekomfort der Messvorrichtung für einen Patienten erhöht. Vorzugsweise ist auch die Abdeckung flexibel ausgebildet, so dass sie sich der Kontur der Oberseite von Sensormatte und Sensoreinheit anpassen kann. Da gemäss der Erfindung die Abdeckung erst auf die Sensormatte mit der darin aufgenommenen Sensoreinheit angebracht wird, wenn diese z. B. mit der Haftlage auf der Körperoberfläche befestigt ist, kann die Abdeckung sich deren Form anpassen, wie sie von der Körperoberfläche vorgegeben wurde.

In einer Ausführungsform kann die Sensormatte am Aussenumfang mehrere Einkerbungen und/oder Dünnstellen aufweisen. Vorzugsweise sind die Einkerbungen und/oder Dünnstellen rund um die Sensormatte vorgesehen. Die Einkerbungen und/oder Dünnstellen können z. B. in regelmässigen Abständen vorliegen. In Bereichen in welche eine besondere Biegsamkeit der Sensormatte notwendig ist, können die Einkerbungen und/oder Dünnstellen dichter vorgesehen werden. Die Einkerbungen können z. B. als Schlitze oder rundliche Einschnitte vorgesehen werden. Die Dünnstellen können durch Bereiche gebildet werden, in welchen die Sensormatte dünner ist als in übrigen Bereichen. Zum Beispiel können die Dünnstellen durch Materialaussparungen auf der Oberseite der Sensormatte erreicht werden. Die Unterseite der Sensormatte vorzugsweise eine einheitlich plan Fläche, um eine sichere Auflage auf der Körperoberfläche zu gewährleisten. Die Einkerbungen und Dünnstellen ermöglichen ein leichtes Anpassen der Sensormatte an die Kontur einer Körperoberfläche.

In einer weiteren Ausführungsform der Messvorrichtung nach der vorliegenden Erfindung ist auf der unteren Seite der Sensormatte zumindest auf der Auflagefläche wenigstens bereichsweise eine lösbare, bzw. auswechselbare, Befestigungslage in Form einer transparenten Haftlage angeordnet, wie oben erwähnt. Vorzugweise erstreckt sich die Haftlage über die gesamte Auflagefläche und besonders bevorzugt über die gesamte Unterseite der Messvorrichtung, d. h. über die gesamte Auflagefläche der Sensormatte und die Unterseite der Aufnahme. Die Haftlage ist derart ausgebildet, dass sie mit einer Seite an Sensormatte und Sensoreinheit und mit der anderen Seite auf der Körperoberfläche haftet. Die Haftlage ist vorzugsweise von der Sensormatte abnehmbar, bzw. entfernbar oder erneuerbar, ausgebildet. Die Haftlage kann z. B. als eine mit einem Haftmittel präparierte Gewebelage oder einer präparierten Folie gegeben sein. Zusätzlich zur Haftlage kann auch ein Gel, eine Creme, ein sprühbares Mittel oder dergleichen vorgesehen werden, welche die Hafteigenschaft ermöglichen oder verbessern. Vorzugsweise wird die Haftlage nach einer durchgeführten Messung von der Messvorrichtung entfernt und für eine erneute Messung wird eine neue, bzw. weitere Haftlage zwischender Unterseite von Sensormatte und Sensoreinheit und Körperoberfläche vorgesehen. Es kann aber auch eine zweite Haftlage über der ersten Haftlage vorgesehen werden, so dass die Messvorrichtung mit der zweiten Haftlage erneut auf der Körperoberfläche befestigt werden kann.

In einer weiteren Ausführungsform einer Messvorrichtung nach der vorliegenden Erfindung ist eine Kontrolleinheit vorgesehen, welche eine Hintergrundbeleuchtung in Richtung der Körperoberfläche zwischen einzelnen Laserimpulsen von Laserlichtquellen der Sensoranordnung erfasst. Die Kontrolleinheit kann intern innerhalb der Aufnahme der Sensoreinheit vorgesehen sein. Alternativ kann die Kontrolleinheit als externe Einheit vorgesehen sein, welche z. B. über die Leitungen der Sensoreinheit mit den Messflächen verbunden ist. Bei dieser Ausführungsform können die Messflächen sowohl zur Messung der Parameter des Körpergewebes als auch zur Messung der Hintergrundbeleuchtung dienen. Vorzugsweise wird während der Aussendung von Laserimpulsen, bzw. während des Empfangens von am Körpergewebe reflektiertem oder gestreutem Licht, d. h. dem zu messenden Licht, das gesamte auf die Messflächen einfallende Licht ermittelt. Es wird also sowohl die Hintergrundbeleuchtung als auch das zu messende Licht erfasst. Zur Ermittlung des zu messenden Lichts und damit zur Bestimmung der Messparameter wird ein vor dem Aussenden eines Laserimpulses gemessener Wert der Hintergrundbeleuchtung von dem insgesamt einfallenden Licht abgezogen.

Ferner wird die ermittelte Hintergrundbeleuchtung verwendet, um eine Not-Ausschaltung für die Messvorrichtung zu realisieren. Sobald der Wert der Hintergrundbeleuchtung einen vorbestimmten Maximalwert überschreitet werden alle Lichtquellen abgeschaltet, um sicherzustellen, dass diese keine Gefahr darstellen, falls die Messvorrichtung von der Körperoberfläche absichtlich oder unabsichtlich entfernt wird.

Bei noch einer weiteren Ausführungsform einer Messvorrichtung nach der vorliegenden Erfindung wird zum Einen durch die Kontrolleinheit das von der Lichtquelle abgestrahlte Licht ermittelt. Zum Anderen kann mittels einer dazu unabhängigen zusätzlichen Lichtauskopplung das abgestrahlte Licht erfasst. Durch diese beiden Massnahmen kann eine Emission zu hoher Lichtenergie vermieden werden. Beispielsweise kann über den Laserdiodenstrom die Laserleistung jederzeit nachgeregelt werden.

Die beiden vorgenannten Ausführungsformen einer Messvorrichtung stellen unabhängig von einer oben beschriebenen Anordnung der Sensoreinheit und der Sensormatte eine vorteilhafte Weiterentwicklung bekannter Messgeräte zu Messung zerebraler Parameter dar. Es bleibt daher vorbehalten, auf diese Aspekte der vorliegenden Erfindung eine eigene Patentanmeldung zu richten.

Nachfolgend wird ein Verfahren zur Bereitstellung einer Messvorrichtung zur nicht-invasiven Messung von Parametern eines Körpergewebes nach der vorliegenden Erfindung beschrieben. Vorzugsweise wird dabei eine Messvorrichtung der oben beschriebenen Art verwendet. Zur Bereitstellung der Messvorrichtung wird eine Sensoreinheit in eine Aussparung einer Sensormatte eingesetzt. Anschliessend wird eine Haftlage zumindest auf der unteren Seite der Sensormatte angebracht. Die Einheit aus Sensormatte und Sensoreinheit wird mit der Haftlage auf der Körperoberfläche befestigt. Anschliessend an die Befestigung der Einheit aus Sensormatte und Sensoreinheit auf der Körperoberfläche wird die Aussparung durch Anbringen einer Abdeckung auf einer Oberseite der Sensormatte (8) abgedeckt, bzw. abgeschlossen.

Die Sensormatte, die Sensoreinheit und die Abdeckung sind von einander lösbar, so dass die Abdeckung von Sensormatte und Sensoreinheit abgenommen werden kann, ohne diese von der Körperoberfläche entfernen zu müssen. Ferner kann die Sensoreinheit aus der Aussparung der Sensormatte entnommen werden, ohne die Sensormatte von der Körperoberfläche abzunehmen. Dadurch kann die Sensoreinheit vom Patienten entfernt werden, z. B. wenn dieser anderen Messverfahren oder Anwendungen zur Verfügung stehen soll. Die Sensormatte kann dabei auf der Körperoberfläche verbleiben und markiert den Ort der Messung. Wird wieder eine Sensoreinheit in die Sensormatte eingesetzt, ist sichergestellt, dass die Parametermessung an der gleichen Stelle fortgesetzt werden kann. Demnach kann nach einem Abnehmen der Abdeckung die Sensoreinheit aus der Sensormatte entnommen werden, während die Sensormatte auf der Körperoberfläche befestigt bleibt. Anschliessend kann wieder eine Sensoreinheit in die auf der Körperoberfläche verbliebene Sensormatte eingesetzt und die Aussparung mit einer weiteren Abdeckung verschlossen werden.

Die Haftlage ist vorzugsweise auf einer unteren Seite der Sensormatte vorgesehen und wird zumindest teilweise auf einer Auflagefläche der Sensormatte zur Auflage auf der Körperoberfläche angebracht. Dabei umgibt die Auflagefläche die Sensoreinheit zumindest teilweise. Vorzugsweise wird die Haftlage über die gesamte Unterseite der Sensormatte und der Sensoreinheit aufgebracht, während die Auflagefläche die Sensoreinheit vollständig umgibt. Wird die Sensormatte mit der eingesetzten Sensoreinheit mit der Haftlage auf der Körperoberfläche angehaftet, können sich Sensormatte und Sensoreinheit der Körperoberfläche anpassen. Beim anschliessenden Anbringen der Abdeckung passt sich diese wiederum der Oberflächenkontur der Oberseite von Sensormatte und Sensoreinheit an. Die Abdeckung kann z. B. durch eine Haft- oder lösbare Klebeschicht befestigt werden.

Anschliessend werden die Parameter des Körpergewebes mittels der Sensoreinheit erfasst. Bei dem erfindungsgemässen Verfahren wird durch die Abdeckung über die gesamte untere Fläche der Sensoreinheit ein gleichmässiger Druck und/oder ein über die Fläche konstanter Druck zwischen Sensoreinheit und Körperoberfläche generiert. Somit werden an der unteren Fläche der Sensoreinheit angeordnete Elemente einer Sensoranordnung, wie etwa Messflächen und Lichtausgänge, mit konstantem Druck auf die Körperoberfläche gepresst.

Bei dem Verfahren nach der vorliegenden Erfindung wird ein zuverlässiger homogener Kontakt zwischen der Sensoreinheit und der Körperoberfläche hergestellt, so dass die Qualität der ermittelten Messwerte verbessert wird.

Werden keine weiteren Messungen zerebraler Parameter vorgesehen wird letztlich auch die Sensormatte und die Haftschicht von der Körperoberfläche entfernt. Die Sensormatte wird dann entsorgt oder gereinigt und die Sensoreinheit kann mit einer weiteren Sensormatte für eine weitere Parametermessung zur Verfügung gestellt werden. Grundsätzlich kann natürlich auch die Messvorrichtung als Ganzes abgenommen werden, d. h. Sensormatte, Sensoreinheit, Abdeckung und Haftlage als eine Einheit.

### Kurze Beschreibung der Zeichnungen

Eine bevorzugte Ausführungsform der Erfindung wird im Folgenden anhand der Zeichnungen dargestellt, die lediglich zur Erläuterung dienen und nicht einschränkend auszulegen sind. Aus den Zeichnungen offenbar werdende Merkmale der Erfindung sollen einzeln und in jeder Kombination als zur Offenbarung der Erfindung gehörend betrachtet werden. In den Zeichnungen zeigen:
- Fig. 1:: eine schematische Darstellung als Schnittansicht durch eine Messvorrichtung nach der vorliegenden Erfindung,
- Fig. 2:: eine Messvorrichtung nach der vorliegenden Erfindung mit einer Sensoreinheit und einer Sensormatte in einer dreidimensionalen Darstellung,
- Fig. 3:: eine Detailansicht der Sensoreinheit der Messvorrichtung nach Figur 2,
- Fig. 4:: eine Aufnahme einer Sensoreinheit nach Figur 3 und
- Fig. 5:: eine Detailansicht der Sensormatte der Messvorrichtung nach Figur 2.

In der folgenden Beschreibung einer Messvorrichtung nach der vorliegenden Erfindung soll eine Unterseite als eine einer Körperoberfläche zugewandte Seite und eine Oberseite als eine der Unterseite gegenüberliegende Seite verstanden werden. Dabei sind Ober- und Unterseiten im Wesentlichen zumindest annähernd parallel zu einer Körperoberfläche. Umfangsflächen stehen im Wesentlichen senkrecht zu Ober- und Unterseiten, bzw. zur Körperoberfläche. Eine Dicke wird in einer Richtung senkrecht zu einer Ober-, bzw. Unterseite und eine Breite parallel zu diesen Seiten angegeben.

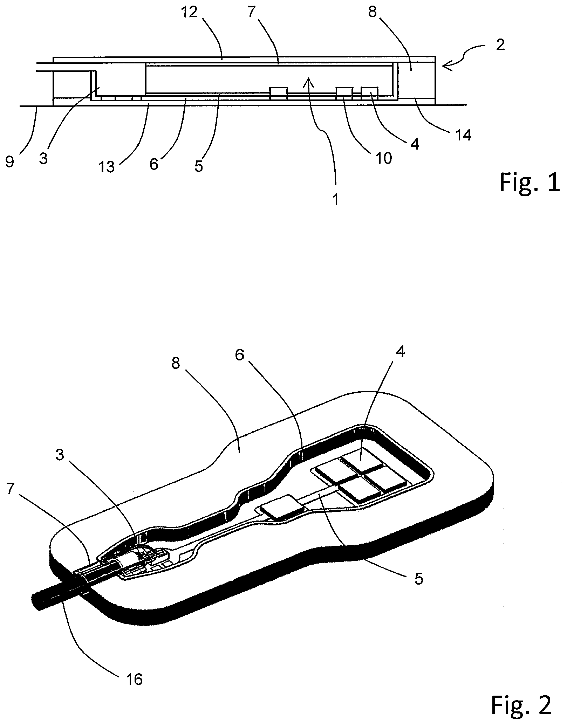

In Figur 1 ist in schematischer Weise der Aufbau einer Messvorrichtung nach der Erfindung zur nicht-invasiven Messung von Parametern eines Körpergewebes mit einer Sensoreinheit 1 und einer Sensormatte 8 zum lösbaren Aufbringen der Messvorrichtung auf einer Körperoberfläche 9 gezeigt.

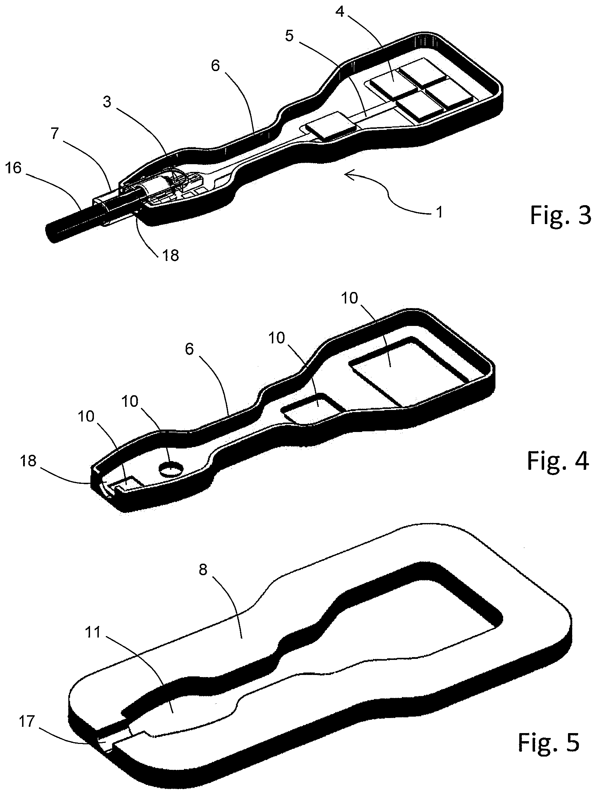

In dieser Ausführungsform besteht die Messvorrichtung im wesentlichen aus zwei Einheiten: einer wiederverwendbare Sensoreinheit 1 und einer Trägereinheit 2. Die wiederverwendbare Sensoreinheit 1 weist eine Sensoranordnung mit einer Lichtquelle 3, dazu beabstandete Messflächen 4 und optische Leitungen 5 auf. Weitere Elemente können in der Sensoranordnung vorgesehen werden. Die Lichtquelle 3 besteht aus vier verschiedenen Laserdioden, die im nahinfraroten Bereich (NIRS) jeweils mit unterschiedlicher Wellenlänge Licht emittieren und zur Messung von Parametern vorzugsweise zeitlich gestaffelt ein- und ausgeschalten werden. Als Messflächen 4 werden z. B. Fotodioden verwendet. Die Sensoranordnung ist innerhalb einer länglichen, flachen Aufnahme 6 mit definierter Aussenkontur untergebracht, wobei die Aussenkontur auf die einzelnen Elemente der Sensoranordnung abgestimmt ist. Wie aus Figur 3 ersichtlich ist, ist die Aufnahme 6 an einer Oberseite offen und weist auf einer Unterseite eine Grundfläche mit mehreren Öffnungen 10 u. a. für den Durchgang von Licht auf. Die Grundfläche dient als Sensorfläche 15 und ist der Körperoberfläche 9 zugewandt. Die Oberseite ist mit einer lichtdichten Verschlussabdeckung 7 verschlossen. Die Aufnahme 6 ist flexibel ausgebildet, um sich der Kontur der Körperoberfläche 9 anpassen zu können. Die Aufnahme 6 ist daher biegsam und kann z. B. eine konkave oder konvexe Form annehmen.

Die Trägereinheit 2 ist als einmal verwendbare Einheit vorgesehen und wird nach einmaligem Gebrauch entsorgt. Alternativ kann die Trägereinheit auch mehrfach verwendet werden. Die Trägereinheit 2 weist eine Sensormatte 8 auf, die z. B. aus Schaum gefertigt ist. Die Sensormatte 8 weist eine innere Aussparung 11 auf, die einen Durchgang durch die Sensormatte 8 bildet und dem Umfang der Aussenkontur der Aufnahme 6 entspricht. An einer Oberseite der Sensormatte 8 ist über die gesamte Oberfläche eine Abdeckung 12 angebracht. Auf der Unterseite weist die Sensormatte 8 eine Auflagefläche 14 zur Auflage auf der Körperoberfläche 9 auf. Die Auflagefläche 14 ist auf Grund der Ausgestaltung der Aussparung 11 im wesentlichen ringförmig und umgibt die Sensoreinheit 1 vollumfänglich, wenn diese in die Sensormatte 8 eingesetzt ist. Alternative könnte die Auflagefläche die Sensoreinheit 1 auch nur bereichsweise umgeben. Es ist jedoch sicherzustellen, dass die Sensoreinheit 1 sicher in der Aussparung 11 gehalten wird. Die Sensormatte 8 steht seitlich mit einer von Breite von durchschnittlich 5 mm über den Umfang der Aufnahme 6 hinaus. Die Breite bestimmt die Grösse der Auflagefläche 14 und somit die Auflage auf der Körperoberfläche zur Befestigung der Messvorrichtung. Auf der Auflagefläche 14 ist eine abnehmbare Haftlage 13 angeordnet. Ferner erstreckt sich die Haftlage 13 über die Sensorfläche 15 der Sensoreinheit 1. Die Abdeckung 12 wird mittels einer Klebeschicht lösbar auf der oberen Oberfläche der Sensormatte 8 befestigt, welche die Aufnahme 6 umgibt. Die Klebeschicht kann dabei nur über der Sensormatte oder über Sensormatte und Sensoreinheit vorgesehen werden.

Die Abdeckung 12 wirkt als Anschlag für die Sensormatte 8, wenn die Messvorrichtung mit der Unterseite von Aufnahme 6 und Sensormatte 8 auf der Körperoberfläche 9 fest sitzt, so dass ein konstanten und gleichmässigen Druck zwischen wiederverwendbarer Sensoreinheit 1 und Körperoberfläche 9 generiert. Dabei kann sich die Aufnahme 6 auf Grund des Anschlags an der Abdeckung 12 nicht innerhalb der Aussparung 11 in Richtung der Abdeckung aus der Sensormatte 8 bewegen.

In der Aufnahme 6 ist eine Kontrolleinheit vorgesehen, welche u. a. die Funktionen der Sensoranordnung kontrolliert. Die Kontrolleinheit misst über die Messflächen 4 automatisch eine Hintergrundbeleuchtung vor der Unterseite der Messvorrichtung. Dabei wird die Hintergrundbeleuchtung jeweils zwischen den Laserlichtpulsen der Lichtquelle 3 gemessen. Während den Laserlichtpulsen wird das Gesamtlicht der Lichtquelle 3 gemessen und anschliessend wird die vorher gemessene Hintergrundbeleuchtung davon subtrahiert. Löst sich eine Messvorrichtung oder fällt sie ab, verändert sich die Hintergrundbeleuchtung und alle Laserdioden der Lichtquelle 3 werden automatisch ausgeschaltet, sobald die Hintergrundbeleuchtung einen bestimmten Wert überschreitet. Ferner wird in der Kontrolleinheit der elektrische Strom durch die Laserdioden gemessen. Zusätzlich wird über eine weitere optische Auskopplung (nicht gezeigt) das tatsächlich abgestrahlte Licht ermittelt. Durch den gemessenen elektrischen Strom und die Ermittlung des abgestrahlten Lichts kann eine Emission zu hoher Laserlichtenergie vermieden werden. Über den Laserdiodenstrom kann die Laserleistung jederzeit nachgeregelt werden. Die Kontrolleinheit kann bezüglich der optischen Leistung der Laserdiode und der Linearität nach der Montage kalibriert werden. Die Kalibrierung kann regelmässig wiederholt werden, falls erforderlich. Die Leitungen 5 innerhalb der Aufnahme 6 weisen eine EMI-Abschirmung auf, um eine Verfälschung der Messwerte zu vermeiden.

In Figur 2 ist eine dreidimensionale Ansicht der Messvorrichtung gezeigt, wobei die Sensoreinheit 1 in die Sensormatte 8 eingesetzt ist. Die Aussenkontur der Aufnahme 6 liegt am Innenumfang der Sensormatte 8 an. Ein Zugang 16 ragt seitlich aus der Aufnahme 6 und der Sensormatte 8 heraus. Der Zugang 16 wird in der Sensormatte in einer Vertiefung 17 gelagert, so dass die Auflagefläche nicht unterbrochen ist (siehe Figur 5). Der Zugang dient als Zu- und Ableitung elektrischer oder optischer Signal, der Stromversorgung und der Verbindung mit einer externen Steuer- und Verarbeitungseinheit und/oder einer Kontrolleinheit. Die Aufnahme 6 schliesst auf der Oberseite und der Unterseite im Wesentlichen plan mit der Oberseite und der Unterseite der Sensormatte 8 ab, so dass eine Abdeckung 12 mit einer planen Unterseite auf der Oberseite befestigt werden kann.

In Figur 3 ist in schematischer dreidimensionaler Darstellung, wie oben erwähnt, eine Sensoreinheit 1 nach der vorliegenden Erfindung gezeigt. In der Sensoreinheit 1 ist eine Sensoranordnung untergebracht, die eine Lichtquelle 3, Messflächen 4 und Leitungen 5 aufweist. Die Aufnahme 6 ist als eine Art flaches Gehäuse oder Schale mit einer Grundfläche und einer Umfangswandung ausgebildet. Die Umfangswandung weist einen Durchgang 18 für den Zugang 16 auf. Der Durchgang 18 ist nach oben offen, so dass der Zugang 16 einfach eingelegt werden kann. Die Verschlussabdeckung 7 erstreckt sich über den Innenraum und teilweise über den Zugang 17.

In Figur 4 ist die Aufnahme 6 ohne weitere Elemente gezeigt. Die Grundfläche, welche die Basis für die Sensorfläche 15 bildet, weist mehrere Öffnungen 10 auf, über welchen die Elemente der Sensoranordnung vorgesehen werden, beispielsweise die Messflächen 4 und die Lichtquelle 3. In Figur 5 ist die Trägereinheit 2 in Form der Sensormatte 8 ohne eingesetzte Sensoreinheit 1 gezeigt. Die Sensormatte 8 ist als längliche flache Matte ausgebildet. Daraus wird die Innenkontur der Aussparung 11 ersichtlich, die der Aussenkontur der Aufnahme 6 entspricht. Die Breite der Sensormatte 8 vom Rand der Aussparung 11 bis zur äusseren Umfangsseite ist rings um die Aussparung zumindest annähernd gleich mit geringen Abweichungen in Bereichen, in welchen die Aussenkontur der Aufnahme 6 stark variiert. Die Anordnung der Vertiefung 17 in der Sensormatte 8 korrespondiert mit der Anordnung des Durchgangs 18 in der Aufnahme 6. Die Sensormatte 8 ist als Schaumelement vorgesehen, das in grossen Stückzahlen in einfacher und kostengünstiger Weise hergestellt werden kann. Die Trägereinheit 2 kann daher als Einweg-Einheit vorgesehen werden.

### BEZUGSZEICHEN

- 1: Sensoreinheit
- 2: Trägereinheit
- 3: Lichtquelle
- 4: Messfläche
- 5: Leitung
- 6: Aufnahme
- 7: Verschlussabdeckung
- 8: Sensormatte
- 9: Körperoberfläche
- 10: Öffnung
- 11: Aussparung
- 12: Abdeckung
- 13: Haftlage
- 14: Auflagefläche
- 15: Sensorfläche
- 16: Zugang
- 17: Vertiefung
- 18: Durchgang

## Patentansprüche

1. Messvorrichtung zur nicht-invasiven Messung von Parametern eines Körpergewebes mit einer Abdeckung (12), einer Sensoreinheit (1) und einer Sensormatte (8) zum lösbaren Aufbringen der Messvorrichtung auf einer Körperoberfläche, wobei die Sensoreinheit (1) eine Aufnahme (6) aufweist, die in ihrem Innenraum eine Sensoranordnung aufnimmt, wobei die Aufnahme (6) eine Sensorfläche (15) in Richtung der Körperoberfläche aufweist, und wobei die Abdeckung (12), die Sensoreinheit (1) und die Sensormatte (8) voneinander lösbar sind,
- die Sensormatte (8) eine innere Aussparung (11) zur Aufnahme der Sensoreinheit (1) und eine zumindest teilweise um die Sensoreinheit (1) umlaufende untere Auflagefläche (14) zur Auflage auf der Körperoberfläche (9) aufweist, und
- die Abdeckung (12) über eine obere Seite von Sensoreinheit (1) und Sensormatte (8) zum Verschliessen der Aussparung (11) während einer Messung von Parametern vorgesehen ist,
**dadurch gekennzeichnet, dass** die Aussparung einen Durchgang durch die Sensormatte bildet mit einer Form, welche auf die äußere Form der Aufnahme abgestimmt ist, so dass die Sensoreinheit innerhalb der Aussparung formschlüssig aufnehmbar ist.

2. Messvorrichtung nach Anspruch 1, **dadurch gekennzeichnet, dass** die Fläche der Abdeckung (12) im Wesentlichen dem Ausmass der Oberseite der Sensormatte (8) entspricht.

3. Messvorrichtung nach einem der vorhergehenden Ansprüche, **dadurch gekennzeichnet, dass** eine transparente Haftlage (13) über eine untere Seite von Sensoreinheit (1) und Sensormatte (8) zur Befestigung der Messvorrichtung auf einer Körperoberfläche (9) vorgesehen ist.

4. Messvorrichtung nach einem der vorhergehenden Ansprüche, **dadurch gekennzeichnet, dass** die Aufnahme (6) zur Oberseite hin zumindest teilweise offen ist und mit einer lichtdichten Verschlussabdeckung (7) verschlossen ist.

5. Messvorrichtung nach Anspruch 4, **dadurch gekennzeichnet, dass** die Verschlussabdeckung (7) von einer Versiegelung gebildet wird, welche die Sensoranordnung im Innenraum der Aufnahme (6) versiegelt.

6. Messvorrichtung nach einem der Ansprüche 3 bis 5, **dadurch gekennzeichnet, dass** die Aufnahme (6) an einer Innenseite der Abdeckung (12) und einer Innenseite der Haftlage (13) anschlägt.

7. Messvorrichtung nach einem der vorhergehenden Ansprüche, **dadurch gekennzeichnet, dass** die Sensormatte (8), die Aufnahme (6) und die Abdeckung (12) zumindest teilweise flexibel ausgebildet sind.

8. Messvorrichtung nach einem der vorhergehenden Ansprüche, **dadurch gekennzeichnet, dass** die Sensormatte (8) am Aussenumfang mehrere Einkerbungen und/oder Dünnstellen aufweist.

9. Messvorrichtung nach einem der vorhergehenden Ansprüche, **dadurch gekennzeichnet, dass** sie als Bausatz mit mehreren zusammen zu setzenden Elementen vorliegt, wobei die Sensoreinheit (1) als wiederverwendbares Element und die Sensormatte (8) und die Abdeckung (12) als Einweg-Elemente vorgesehen sind.

10. Messvorrichtung nach einem der vorhergehenden Ansprüche, **dadurch gekennzeichnet, dass** sie eine Kontrolleinheit aufweist, welche eine Hintergrundbeleuchtung in Richtung der Körperoberfläche (9) zwischen Laserimpulsen von Laserlichtquellen der Sensoranordnung erfasst.

11. Verfahren zur Bereitstellung einer Messvorrichtung zur nicht-invasiven Messung von Parametern eines Körpergewebes gemäss folgenden Schritten:
- Einsetzen einer Sensoreinheit (1), umfassend eine in eine Aufnahme (6) eingesetzte Sensoranordnung, in eine innere Aussparung (11) einer Sensormatte (8), wobei die Aussparung (11) einen Durchgang durch die Sensormatte (8) bildet mit einer Form, welche auf eine äussere Form der Aufnahme (6), abgestimmt ist, so dass die Sensoreinheit (1) innerhalb der Aussparung formschlüssig aufnehmbar ist,
- Anbringen einer Haftlage (13) zumindest auf der unteren Seite der Sensormatte (8),
- Befestigen der Einheit aus Sensormatte (8) und Sensoreinheit (1) auf der Körperoberfläche (9) mit der Haftlage (13) und
- Abdecken der Aussparung (11) durch Anbringen einer Abdeckung (12) auf einer Oberseite der Sensormatte (8) anschliessend an die Befestigung der Einheit aus Sensormatte (8) und Sensoreinheit (1) auf der Körperoberfläche (9).

12. Verfahren nach Anspruch 11, wobei durch die Abdeckung (12) ein gleichmässiger und/oder konstanter Druck zwischen Sensoreinheit (1) und Körperoberfläche (9) generiert wird.

13. Verfahren nach einem der Ansprüche 11 oder 12, wobei die Sensoreinheit (1) nach Abnehmen der Abdeckung (12) aus der Sensormatte (8) entnommen wird, während die Sensormatte (8) auf der Körperoberfläche (9) befestigt bleibt und anschliessend wieder eine Sensoreinheit (1) in die auf der Körperoberfläche (9) verbliebene Sensormatte (8) eingesetzt und die Aussparung (11) mit einer weiteren Abdeckung (12) verschlossen wird.

14. Verfahren nach einem der Ansprüche 11 bis 13, wobei die Sensoreinheit (1) nach einer Durchführung einer Parametermessung von der Sensormatte (8) entfernt wird, die Sensormatte (8) entsorgt oder gereinigt wird und die Sensoreinheit (1) mit einer weiteren Sensormatte (8) und einer weiteren Abdeckung (12) für eine weitere Parametermessung zur Verfügung gestellt wird.

## Claims

1. Measuring device for non-invasive measurement of parameters of a bodily tissue, with a covering (12), a sensor unit (1) and a sensor pad (8) for detachable placement of the measuring device on a body surface, whereby the sensor unit (1) has a receptacle (6), the interior space of which accommodates a sensor arrangement, the receptacle (6) having a measuring surface (15) in the direction of the body surface, and whereby the covering (12), the sensor unit (1), and the sensor pad (8) are detachable from one another,
- the sensor pad (8) has a cutout (11) for accommodating the sensor unit (1) and a lower contact surface (14), at least partially surrounding the sensor unit (1), for placement on the body surface (9),
- the covering (12) is provided for closing the cutout (11) over an upper side of sensor unit (1) and sensor pad (8) during a measurement of parameters,
**characterized in that** the cutout forms a passage through the sensor pad with a shape that matches the outer shape of the receptacle so that the sensor unit is receivable inside the cutout in a formfitting way.

2. Measuring device according to claim 1, **characterized in that** the surface of the covering (12) corresponds substantially to the size of the upper side of the sensor pad (8).

3. Measuring device according to one of the preceding claims, **characterized in that** a transparent adhesive layer (13) is provided over a lower side of sensor unit (1) and sensor pad (8) for fixing the measuring device to a body surface (9).

4. Measuring device according to one of the preceding claims, **characterized in that** the receptacle (6) is at least partially open toward the upper side and is closed with a lightproof closing cover (7).

5. Measuring device according to claim 4, **characterized in that** the closing cover (7) is formed by a sealing which seals the sensor arrangement in the interior space of the receptacle (6).

6. Measuring device according to one of the claims 3 to 5, **characterized in that** the receptacle (6) abuts on an inner side of the covering (12) and an inner side of the adhesive layer (13).

7. Measuring device according to one of the preceding claims, **characterized in that** the sensor pad (8), the receptacle (6) and the covering (12) are designed at least partially flexible.

8. Measuring device according to one of the preceding claims, **characterized in that** the sensor pad (8) has on its outer periphery a plurality of indentations and/or thin places.

9. Measuring device according to one of the preceding claims, **characterized in that** it is provided as a kit with a plurality of elements to be put together, the sensor unit (1) being provided as reusable element, and sensor pad (8) and the covering (12) being provided as disposable articles.

10. Measuring device according to one of the preceding claims, **characterized in that** it has a control unit which registers a background lighting in direction of the body surface (9) between laser pulses of laser light sources of the sensor arrangement.

11. Method of preparing a measuring device for non-invasive measurement of parameters of a bodily tissue according to the following steps:
- inserting a sensor unit (1), comprising a sensor arrangement inserted into a receptacle (6), into an inner cutout (11) of a sensor pad (8), whereby the cutout (11) forms a passage through the sensor pad (8) with a shape that matches an outer shape of the receptacle (6) so that the sensor unit (1) is receivable inside the cutout in a formfitting way
- attaching an adhesive layer (13) at least to the lower side of the sensor pad (8),
- fixing the unit of sensor pad (8) and sensor unit (1) to the body surface (9) with the adhesive layer (13) and
- covering the cutout (11) by putting a covering (12) on an upper side of the sensor pad (8) subsequent to the attachment of the unit of sensor pad (8) and sensor unit (1) to the body surface (9).

12. Method according to claim 11, whereby a steady and/or constant pressure is generated between sensor unit (1) and body surface (9) by the covering (12).

13. Method according to one of the claims 11 or 12, whereby, after removal of the covering (12), the sensor unit (1) is taken out of the sensor pad (8) while the sensor pad (8) remains attached to the body surface (9) and then another sensor unit (1) is inserted into the sensor pad (8) remaining on the body surface (9) and the cutout (11) is closed with a further covering (12).

14. Method according to one of the claims 11 to 13, whereby, after a carrying out of a parameter measurement, the sensor unit (1) is removed from the sensor pad (8), the sensor pad (8) is disposed of or is cleaned, and the sensor unit (1) with another sensor pad (8) and a further covering (12) is made available for another parameter measurement.

## Revendications

1. Dispositif de mesure pour la mesure non-invasive de paramètres d'un tissu corporel, comprenant un couvercle (12), une unité de détection (1) et un bloc détecteur (8) pour le placement amovible du dispositif de mesure sur une surface corporelle, l'unité de détection (1) possédant un réceptacle (6), logeant un dispositif de détection dans son espace intérieur, le réceptacle (6) possédant une surface de mesure (15) dans la direction de la surface corporelle, et le couvercle (12), l'unité de détection (1), et le bloc détecteur étant mutuellement détachables les uns des autres,
- le bloc détecteur (8) possédant un renfoncement (11) interne accueillant l'unité de détection (1) et une surface de revêtement inférieure (14), surmontant au moins partiellement l'unité de détection (1), pour recouvrir la surface corporelle (9),
- le couvercle (12) est prévu pour refermer le renfoncement (11) via un côté supérieur de l'unité de détection (1) et du bloc de détecteur (8) pendant une mesure de paramètres,
**caractérisé en ce que** le renfoncement forme un passage à travers le bloc détecteur selon une forme qui correspond à la forme externe du réceptacle de telle sorte que l'unité de détection peut être logée à l'intérieur du renfoncement d'une manière adaptée à sa forme.

2. Dispositif de mesure selon la revendication 1, **caractérisé en ce que** la taille de la surface du couvercle (12) correspond substantiellement à celle de la face supérieure du bloc détecteur (8).

3. Dispositif de mesure selon l'une des revendications précédentes, **caractérisé en ce qu'**une couche adhésive transparente (13) est prévue sur la face inférieure de l'unité de détection (1), et un bloc détecteur (8) pour la fixation du dispositif de mesure à une surface corporelle (9).

4. Dispositif de mesure selon l'une des revendications précédentes, **caractérisé en ce que** le réceptacle (6) est au moins partiellement ouvert en direction du côté supérieur et est refermé par un couvercle de fermeture opaque (7).

5. Dispositif de mesure selon la revendication 4, **caractérisé en ce que** le couvercle de fermeture (7) est formé par un scellé qui scelle le dispositif de détection dans l'espace intérieur du réceptacle (6).

6. Dispositif de mesure selon l'une des revendications 3 à 5, **caractérisé en ce que** le réceptacle (6) jouxte un côté interne du couvercle (12) et un côté interne de la couche adhésive (13).

7. Dispositif de mesure selon l'une des revendications précédentes, **caractérisé en ce que** le bloc détecteur (8), le réceptacle (6) et le couvercle (12) sont conçus au moins partiellement de manière flexible.

8. Dispositif de mesure selon l'une des revendications précédentes, **caractérisé en ce que** le bloc de détecteur (8) a une pluralité d'indentations et/ou d'amincissements sur sa périphérie externe.

9. Dispositif de mesure selon l'une des revendications précédentes, **caractérisé en ce qu'**il est fourni sous la forme d'un kit comprenant une pluralité d'éléments à assembler, l'unité de détection (1) étant fournie comme un élément réutilisable, et le bloc de détecteur (8) et la couvercle (12) étant fournis comme articles jetables.

10. Dispositif de mesure selon l'une des revendications précédentes, **caractérisé en ce qu'**il possède une unité de contrôle qui détecte un éclairage de fond en direction de la surface corporelle (9) entre les impulsions laser des sources de lumière laser du dispositif de détection.

11. Méthode de préparation d'un dispositif de mesure pour la mesure non-invasive de paramètres d'un tissu corporel selon les étapes suivantes :
- l'insertion d'une unité de détection (1), comprenant un dispositif de détection inséré dans un réceptacle (6), dans un renfoncement interne (11) d'un bloc détecteur (8), le renfoncement (11) formant un passage à travers le bloc détecteur (8) selon une forme qui correspond à une forme extérieure du réceptacle (6) de telle sorte que l'unité de détection (1) puisse être réceptionnée à l'intérieur du renfoncement d'une manière adaptée à sa forme,
- la fixation d'une couche adhésive (13) au moins du côté inférieur du bloc détecteur (8),
- la fixation de l'unité de bloc détecteur (8) et de l'unité de détection (1) à la surface corporelle (9) avec la couche adhésive (13) et
- le recouvrement du renfoncement (11) en mettant un couvercle (12) sur un côté supérieur du bloc détecteur (8) après la fixation de l'unité de bloc détecteur (8) et de l'unité de détection (1) à la surface corporelle (9).

12. Méthode selon la revendication 1, une pression stable et/ou constante étant générée entre l'unité de détection (1) et la surface corporelle (9) par le couvercle (12).

13. Méthode selon l'une des revendications 11 ou 12, dans laquelle, après l'enlèvement du couvercle (12), l'unité de détection (1) est retirée du bloc détecteur (8) tandis que le bloc détecteur (8) reste fixé à la surface corporelle (9), et ensuite une autre unité de détection (1) est insérée dans le bloc détecteur (8) restant sur la surface corporelle (9) et le renfoncement (11) est refermé par un couvercle supplémentaire (12).

14. Méthode selon l'une des revendications 11 à 13, dans laquelle, après la réalisation d'une mesure de paramètres, l'unité de détection (1) est retirée du bloc détecteur (8), le bloc détecteur (8) est éliminé ou est nettoyé, et l'unité de détection (1) avec un autre bloc détecteur (8) et un couvercle supplémentaire (12) est mis à disposition pour une autre mesure de paramètre.
